# FASCICULE DE BREVET EUROPEEN

(11) **EP 1 675 810 B1**
(45) Date de publication et mention de la délivrance du brevet: **24.05.2017**
(21) Numéro de dépôt: 04818440.2
(22) Date de dépôt: 11.10.2004
(51) Int. Cl.: C07C 17/42, C07C 21/073, C23G 5/028

(54) **STABILISATION DU TRANS-1,2-DICHLORETHYLENE**
STABILISIERUNG VON TRANS-1,2-DICHLORETHYLEN
STABILISATION OF TRANS-1,2-DICHLOROETHYLENE

(30) Priorité: 24.10.2003 FR 0312434
(43) Date de publication de la demande: 05.07.2006
(73) Titulaire: ARKEMA FRANCE, 92700 Colombes (FR)
(72) Inventeur: LALLIER, Jean-Pierre, F-69720 Saint Bonnet de Mure (FR); BAINS, Jessica, F-49300 Cholet (FR)
(74) Mandataire: Dang, Doris
(86) Numéro de dépôt international: PCT/FR2004/002565
(87) Numéro de publication internationale: WO 2005/047220

(56) Documents cités:
- EP-A1- 0 327 282
- EP-A1- 0 343 761
- GB-A- 1 023 382
- GB-A- 1 083 698
- US-A- 3 043 888
- US-A- 6 153 575

## Description

La présente invention concerne la stabilisation du trans-1,2-dichloréthylène (TDCE) en vue de son utilisation pour le traitement des surfaces solides, plus particulièrement pour le nettoyage, le dégraissage, le défluxage ou le séchage des métaux.

Le 1,2-dichloréthylène est un solvant industriel très utilisé pour le traitement des surfaces solides, par exemple pour le nettoyage de surfaces solides, le dégraissage de pièces métalliques ou le défluxage des circuits imprimés.

Le 1,2-dichloréthylène existe sous deux formes isomériques, le cis-1,2-dichloréthylène et le trans-1,2-dichloréthylène. Les isomères du 1,2-dichloréthylène ont des propriétés chimiques et physiques distinctes. En particulier, le trans-isomère a un point d'ébullition, une densité, une viscosité et une tension de surface plus faibles que ceux du cis-isomère. C'est pourquoi, le trans-1,2-dichloréthylène (que l'on désignera par TDCE) est l'isomère préféré dans les applications de nettoyage par solvant. Les deux isomères du 1,2-dichloréthylène peuvent être séparés par distillation fractionnée. Cependant, au cours du stockage, le TDCE se transforme spontanément en cis-isomère, à moins qu'il ne soit stabilisé, le cis-isomère étant plus stable thermodynamiquement. Le brevet US 6,153,575 décrit l'utilisation de faibles quantités d'hydrazones d'aldéhydes aliphatiques, éventuellement en combinaison avec un époxyde pour inhiber l'isomérisation du TDCE en cis-isomère au cours du stockage.

La stabilité au stockage du TDCE est sa capacité à résister à une transformation ou une décomposition durant une période pouvant aller de plusieurs semaines à plusieurs mois dans des containers de stockage conventionnels à une température qui n'atteint généralement pas plus de 50°C.

Au cours de sa mise en oeuvre comme solvant de traitement de surfaces solides, le TDCE est soumis à d'autres agressions, liées au mode de fonctionnement des machines de traitement, qui peuvent entraîner sa décomposition. Les problèmes engendrés par l'utilisation du TDCE sont donc différents de ceux rencontrés lors du stockage.

Les principales sources d'agression du TDCE lors de son utilisation par exemple dans une machine de dégraissage de pièces métalliques sont : l'air, l'eau, les métaux et la chaleur. En effet, le dégraissage métallique est généralement réalisé en introduisant les pièces à dégraisser dans une machine appropriée au-dessus du niveau liquide d'une cuve contenant le TDCE maintenu à ébullition. Les vapeurs de TDCE se condensent sur les pièces métalliques et le condensat est recyclé de façon répétitive. Dans ces conditions, le TDCE est soumis à différentes réactions telles que l'oxydation par l'air, l'hydrolyse par l'eau provenant de la condensation de la vapeur d'eau de l'atmosphère, une dégradation thermique ou des réactions catalytiques au contact des métaux.

Dans la littérature, différentes solutions ont été proposées pour stabiliser certains hydrocarbures halogénés confrontés aux mêmes sources d'agressions. L'addition de différents stabilisants est préconisée dans le brevet US 3,043,888 pour stabiliser les hydrocarbures halogénés comportant de 1 à 3 carbones, plus particulièrement le trichloéthylène, lors de leur utilisation pour le dégraissage métallique. Certains hydrazones d'aldéhydes sont particulièrement efficaces et peuvent être associés à d'autres composés tels que des composés aromatiques contenant un groupe hydroxyle, des amines, des époxydes organiques, des esters d'acides monocarboxyliques, des alcools ou des composés insaturés. Des composés spécialement efficaces pour inhiber la décomposition catalysée par les métaux du méthylchloroforme sont décrits comme étant le 1,3-dioxolane et ses homologues alkyl ou le 1,4-dioxane (voir les brevets US 4,026,956 ; US 3,251,891 ; US 4,418,231 ; US 2,811,252).

Cependant, on ne trouve pas dans les solutions proposées dans la littérature de système de stabilisation d'un solvant, tel qu'un hydrocarbure halogéné, réunissant toutes les caractéristiques exigées d'un stabilisant. Et en particulier, aucun système complet de stabilisation vis à vis de l'air, de l'eau, des métaux et de la chaleur n'est décrit dans l'art antérieur pour le TDCE.

D'abord, le système de stabilisation a de préférence une action double : une action préventive, plus particulièrement pour inhiber les réactions parasites et une action curative, c'est à dire piéger in situ un effet non désiré. Ensuite, pour faciliter la mise en oeuvre du solvant stabilisé, le système de stabilisation a avantageusement une température d'ébullition voisine de celle du solvant, il est avantageusement présent en phase liquide et en phase vapeur, il est insoluble ou peu soluble dans l'eau et de préférence il ne modifie pas les propriétés du solvant. Afin de ne pas modifier l'étiquetage du solvant relatif aux dangers d'utilisation, la formulation de stabilisation est avantageusement non toxique. Enfin, pour des raisons économiques, la formulation stabilisante est constituée de préférence de produits industriels de faible coût.

Il a maintenant été trouvé que l'ajout dans le TDCE de plusieurs additifs ayant chacun une fonction telle qu'accepteur d'acide, piège à radicaux, base de Lewis ou effet tampon, constitue un système de stabilisation répondant parfaitement aux critères mentionnés ci-dessus et permet d'obtenir une solution stabilisée de TDCE qui fait preuve d'une bonne résistance à la décomposition lors de son utilisation pour le traitement des métaux.

Au cours de l'utilisation prolongée à chaud de la même charge de TDCE, l'eau de l'atmosphère, les graisses, les huiles, les salissures et les fines particules métalliques, surtout lorsque les pièces métalliques sont constituées d'aluminium ou de fer, s'accumulent dans le TDCE et sont à l'origine de différentes réactions qui entraînent la décomposition du TDCE. En effet, le TDCE, du fait de sa structure chimique, peut être soumis principalement à trois types de réactivité qui sont l'ouverture de la double liaison, la réactivité du doublet du chlore et l'obtention de radicaux. La dégradation du TDCE conduit le plus souvent à la formation d'acide chlorhydrique ou de radicaux. En présence de métaux, plus particulièrement en présence d'aluminium ou de fer, l'acide chlorhydrique peut réagir pour former du chlorure d'aluminium (AlCl₃) ou du chlorure ferrique (FeCl₃). qui sont en outre des catalyseurs de la réaction exothermique de condensation du TDCE. L'addition d'agents stabilisants tels que des accepteurs d'acide, des pièges à radicaux, des bases de Lewis ou des agents tampon, permet d'avoir une action préventive et/ou curative sur ces différentes réactions.

Il est décrit une solution stabilisée de trans-1,2 dichloréthylène (TDCE) comprenant comme additifs au moins un accepteur d'acide, au moins un piège à radicaux, au moins une base de Lewis et au moins un composé à effet tampon.

La présente invention a pour objet une solution stabilisée de trans-1,2-dichloréthylène comprenant comme additifs au moins :
- un accepteur d'acide choisi parmi les époxydes organiques,
- un piège à radicaux choisi parmi un alcène, un hétérocycle ou un dérivé phénolique,
- une base de Lewis choisie parmi un acétal, une cétone, un composé nitrosé, un ester d'acide carboxylique ou un éther,
- et un composé à effet tampon choisi parmi la N-méthylmorpholine, la diéthylamine et la triéthylamine.

A titre d'exemples non limitatifs d'époxydes organiques, on peut citer l'oxyde d'éthylène, l'oxyde de propylène, l'oxyde de butylène, le monoxyde de butadiène, le dioxyde de butadiène, l'épichlorhydrine, le glycidol, l'oxyde d'isobutylène, l'isopropylglycidyléther De préférence, on utilisera l'oxyde de propylène, l'oxyde de butylène ou l'isopropylglycidyléther.

Dans le cadre de la présente invention, le piège à radicaux est choisi parmi les alcènes, les hétérocycles et les dérivés phénoliques. Comme exemples non limitatifs d'alcènes, on peut citer le disobutylène, l'amylène, l'isoprène ou le α-méthylstyrène Comme exemples non limitatifs d'hétérocycles, on peut citer des pyrroles, plus particulièrement le N-méthylpyrrole, le 1,4-dioxane, des furanes, plus particulièrement le tétrahydrofurane. Comme exemples non limitatifs de dérivés phénoliques, on peut citer le phénol, le thymol ou l'ionol.

On ne sortirait pas du cadre de la présente invention en utilisant un premier piège à radicaux pour la phase vapeur et un second piège à radicaux pour la phase liquide. Par exemple, l'isoprène peut être utilisé comme piège à radicaux pour la phase vapeur et le diisobutylène comme piège à radicaux pour la phase liquide. Cette combinaison de deux pièges a l'avantage d'être à la fois efficace sur le TDCE liquide et sur le TDCE vapeur, ce qui est nécessaire pour l'application visée.

Les bases de Lewis, composés donneurs de doublets électroniques, permettent de saturer les sites acides de Lewis des métaux et ainsi d'inhiber la réaction de formation de AlCl₃ ou FeCl₃. Les bases de Lewis utilisables dans la solution stabilisée selon l'invention sont de nature très variée, et sont choisies parmi les acétals, les cétones, les composés nitrosés, les esters d'acides carboxyliques et les éthers. Comme acétals, on préférera le méthylal. Comme exemples non limitatifs de cétones, on peut citer l'acétone et la méthyl éthyl cétone. Comme exemples non limitatifs de composés nitrosés, on peut citer le nitrométhane ou le nitroéthane. Comme exemples non limitatifs d'esters d'acides carboxyliques, on peut mentionner le formiate de méthyle, l'acétate de méthyle ou l'acétate d'isopropyle. Comme éther, on préférera le tert-butyl méthyl éther.

Parmi les composés à effet tampon utilisables dans la présente invention, les amines sont plus particulièrement adaptées. Elles permettent d'obtenir un pH suffisamment basique pour limiter la cinétique de dégradation du solvant et maintenir un pH relativement constant. Selon l'invention, le composé à effet tampon est choisi parmi la triéthylamine, la N-méthyl morpholine, la diéthylamine et la N,N-diisopropy lamine.

On ne sortirait pas du cadre de l'invention en utilisant en plus des composés mentionnés ci-dessus, un alcool pour bloquer AlCl₃ ou FeCl₃ après leur formation éventuelle.Les alcools pouvant convenir sont par exemple le méthanol, l'éthanol, le n-butanol ou le tertiobutanol.

D'une manière générale, les additifs présents dans le système de stabilisation ont un point d'ébullition voisin de celui du TDCE. Cependant, on peut choisir des composés un peu moins volatils que le TDCE permettant de stabiliser spécifiquement la phase liquide du TDCE, ou inversement on peut choisir des composés plus volatils pour stabiliser efficacement la phase vapeur du TDCE.

La quantité de chacun des additifs présents dans la solution stabilisée de TDCE peut varier considérablement, mais la teneur de chaque additif dans la solution stabilisée sera de préférence comprise entre 10 et 10 000 ppm, de préférence entre 10 et 1000 ppm. La quantité totale d'additifs devra être suffisante pour inhiber la décomposition du TDCE dans les conditions habituelles d'utilisation pour le traitement des métaux, sans altérer les propriétés du solvant. On considère qu'une teneur globale en additifs inférieure à 50 000 ppm et de préférence inférieure à 5000 ppm permet d'obtenir une solution de TDCE stable qui est à la fois efficace pour le traitement des métaux et la plus économique possible.

Plus particulièrement, une solution stabilisée comprend de 200 ppm à 800 ppm d'un accepteur d'acide, de 100 à 700 ppm d'un piège à radicaux ou d'un mélange de pièges à radicaux, de 10 à 100 ppm d'une base de Lewis et de 10 à 50 ppm d'un composé à effet tampon.

Une solution stabilisée de TDCE selon l'invention comprend de l'oxyde de butylène, du diisobutylène, de l'isoprène, de l'acétone et de la diéthylamine. Plus particulièrement, la solution stabilisée comprend de 200 ppm à 800 ppm, de préférence 530 ppm d'oxyde de butylène, de 50 à 500 ppm, de préférence 200 ppm de diisobutylène, de 50 à 200 ppm, de préférence 100 ppm d'isoprène, de 10 à 100 ppm, de préférence 50 ppm d'acétone et de 10 à 50 ppm, de préférence 10 ppm de diéthylamine.

### Partie expérimentale

Pour évaluer l'efficacité de la stabilisation du TDCE, on utilise différents tests dont le principe est exposé ci-après :
Test d'hydrolyse : Le test consiste à suivre l'évolution du pH de la solution stabilisée à tester additivée de 1% d'eau, au cours du chauffage à reflux pendant 100 heures. Pour cela, on utilise un tricol de 500 ml surmonté d'un thermomètre et d'un réfrigérant de Liebig. La troisième ouverture est équipée d'un septum au travers duquel des prélèvements pourront être effectués à l'aide d'une seringue. 247,5 ml de solution à tester sont introduits dans le ballon avec 2,5 ml d'eau. La solution est mise à chauffer à reflux pendant 100 heures. On effectue des prélèvements d'environ 5 à 10 ml toutes les 2-3 heures et on mesure le pH de la solution prélevée après ajout d'une quantité équivalente d'eau à pH 7. Le test d'hydrolyse est négatif si la variation du pH au cours du temps n'est pas significative.
Test de la rayure (ou test scratch) Il est basé sur la norme ASTM D 2943-96.2000. Il consiste à évaluer la stabilité d'un solvant chloré en présence d'une feuille d'aluminium préalablement rayée. Le test scratch est négatif si aucune réaction corrosive ne se produit

### Test BAM (Bundesanstalt fur Materialprüfung - Berlin)

La stabilisation d'une formulation vis-à-vis de l'aluminium est testée grâce au test BAM qui se divise en plusieurs expériences.

On utilise un tricol de 500 ml surmonté d'un réfrigérant de Liebig. La température de l'échantillon est mesurée par une sonde.

100 ml de solution à tester sont introduits dans le tricol avec 100 ml de toluène, 18 g d'aluminium en poudre et 0,7 g d'AlCl₃ anhydre. Le mélange appelé BAM 1 est chauffé à reflux pendant 2 fois 9 heures. On observe si un changement de coloration ou une réaction exothermique se produit.

Une deuxième expérience est réalisée dans les mêmes conditions expérimentales que précédemment après avoir ajouté au mélange BAM 1, 1 g de stéarate de zinc. C'est le milieu réactionnel BAM 2.

Une troisième expérience est réalisée avec un mélange préparé à partir du mélange BAM 1 additivé cette fois de 10 ml d'acide oléique. Le mélange réactionnel est appelé BAM 3.

Si au cours des tests réalisés sur les 3 mélanges BAM 1, BAM 2 et BAM 3, aucun changement de coloration n'est observé ou s'il n'y a pas eu de réaction exothermique, une expérience dénommée BAM 4 est réalisée. Elle consiste à séparer la solution à tester par distillation en trois fractions de même importance. 100 ml de chaque fraction sont additivés de 100 ml de toluène, 18 g d'aluminium en poudre et 0,7 g d'AlCl₃ anhydre, puis chauffés à reflux pendant 2 fois 9 heures. Le test est négatif si aucune réaction n'est produite pendant le chauffage et si aucun changement de coloration n'apparaît.

Les exemples suivants illustrent l'invention sans la limiter.

### Exemple 1 (comparatif)

On effectue le test d'hydrolyse sur une solution de TDCE de la société PPG industries, qui contient 30 ppm d'oxyde de butylène. On observe une évolution du pH qui passe d'une valeur initiale de 7,17 à une valeur de 7,97 après une durée de 100 heures. Cette solution ne peut donc pas être considérée comme stable.

### Exemple 2

A partir de TDCE de la société PPG industries, on prépare une solution de TDCE stabilisée qui contient 530 ppm d'oxyde de butylène, 300 ppm de diisobutylène, 50 ppm d'acétone et 10 ppm de N-méthylmorpholine.
Le test d'hydrolyse montre que le pH ne subit qu'une faible variation, entre 7,78 et 7,94 sur une durée de 100 heures.
Lors du test de la rayure, aucune réaction n'est observée ; l'aluminium n'est pas attaqué par la solution dans les conditions du test.
Dans les conditions du test BAM, la solution ne réagit dans aucun des milieux réactionnels BAM 1, BAM 2 et BAM 3 puisque aucune réaction exothermique n'est observée. On observe seulement une légère coloration jaune translucide dans le milieu BAM 1 et une coloration verdâtre dans le milieu BAM 2. Selon le test BAM 4, 3 fractions de quantité environ égale ont été recueillies correspondant respectivement aux 3 intervalles de température 39-41°C, 41-41,3°C et 41,3-44°C. Aucune des 3 fractions ne réagit dans le milieu réactionnel. On n'observe pas de réaction exothermique mais uniquement une légère coloration verdâtre pour la première fraction.

### Exemple 3

A partir du TDCE de la société PPG industries, on prépare une solution de TDCE stabilisée qui contient 530 ppm d'oxyde de butylène, 200 ppm de diisobutylène, 100 ppm d'isoprène, 50 ppm d'acétone et 10 ppm de diéthylamine.
Le pH au cours du test d'hydrolyse réalisé sur cette solution reste constant ; le pH initial est 8,75. Après 100 heures, il est égal à 8,79. Cette solution est plus basique que la solution de l'exemple 2. Lors du test de la rayure aucune réaction n'est observée ; l'aluminium n'est pas attaqué par la solution dans les conditions du test.
Dans les conditions du test BAM, la solution ne réagit dans aucun des milieux réactionnels BAM 1, BAM 2 et BAM 3 puisque aucune réaction exothermique n'est observée. On observe seulement une légère coloration jaune translucide dans le milieu BAM 1 et le milieu BAM 2. Selon le test BAM 4, 3 fractions de quantité environ égale ont été recueillies correspondant respectivement aux 3 intervalles de température 36,5-37,8°C, 37,8-38°C et 38-38,2°C. Pour aucune des fractions, on n'observe de réaction exothermique, ni de changement de coloration.

## Revendications

1. Solution stabilisée de trans-1,2-dichloréthylène comprenant comme additifs au moins :
- un accepteur d'acide choisi parmi les époxydes organiques,
- un piège à radicaux choisi parmi un alcène, un hétérocycle ou un dérivé phénolique,
- une base de Lewis choisie parmi un acétal, une cétone, un composé nitrosé, un ester d'acide carboxylique ou un éther,
- et un composé à effet tampon choisi parmi la N-méthylmorpholine, la diéthylamine et la triéthylamine.

2. Solution selon la revendication 1, **caractérisée en ce que** la teneur de chaque additif dans la solution stabilisée est comprise entre 10 et 10000 ppm, de préférence entre 10 et 1000 ppm.

3. Solution selon la revendication 1 ou 2, **caractérisée en ce que** la teneur globale en additifs est inférieure à 50 000 ppm, de préférence inférieure à 5000 ppm.

4. Solution selon l'une quelconque des revendications précédentes, **caractérisée en ce qu'**elle comprend de 200 ppm à 800 ppm d'un accepteur d'acide, de 100 à 700 ppm d'un piège à radicaux ou d'un mélange de pièges à radicaux, de 10 à 100 ppm d'une base de Lewis et de 10 à 50 ppm d'un composé à effet tampon.

5. Solution selon l'une quelconque des revendications précédentes, **caractérisée en ce que** l'époxyde organique est choisi parmi l'oxyde de propylène, l'oxyde de butylène ou l'isopropylglycidyléther et est de préférence l'oxyde de butylène.

6. Solution selon l'une quelconque des revendications précédentes, **caractérisée en ce que** l'alcène est choisi parmi le diisobutylène, l'amylène, l'isoprène ou le α-méthylstyrène.

7. Solution selon l'une quelconque des revendications 1 à 5, **caractérisée en ce que** le piège à radicaux est un hétérocycle choisi parmi le N-méthylpyrrole, le 1,4-dioxane ou le tétrahydrofurane.

8. Solution selon l'une quelconque des revendications 1 à 5, **caractérisée en ce que** le piège à radicaux est un dérivé phénolique choisi parmi le phénol, le thymol ou l'ionol.

9. Solution selon l'une quelconque des revendications précédentes, **caractérisée en ce que** l'acétal est le méthylal.

10. Solution selon l'une quelconque des revendications 1 à 8, **caractérisée en ce que** la cétone est l'acétone ou la méthyl éthyl cétone.

11. Solution selon l'une quelconque des revendications 1 à 8, **caractérisée en ce que** le composé nitrosé est le nitrométhane ou le nitroéthane.

12. Solution selon l'une quelconque des revendications 1 à 8, **caractérisée en ce que** l'ester d'acide carboxylique est le formiate de méthyle, l'acétate de méthyle ou l'acétate d'isopropyle.

13. Solution selon l'une quelconque des revendications 1 à 8, **caractérisée en ce que** la base de Lewis est un éther, de préférence le tert-butyl méthyl éther.

14. Solution selon l'une quelconque des revendications précédentes, **caractérisée en ce que** le composé à effet tampon est la N-méthylmorpholine.

15. Solution selon l'une quelconque des revendications 1 à 13, **caractérisée en ce que** le composé à effet tampon est la diéthylamine.

16. Solution selon l'une des revendications 1 à 4, **caractérisée en ce qu'**elle comprend de l'oxyde de butylène, du diisobutylène, de l'isoprène, de l'acétone et de la diéthylamine.

17. Solution selon la revendication 16, caractérisée en qu'elle comprend de 200 à 800 ppm d'oxyde de butylène, de 100 à 500 ppm de diisobutylène, de 50 à 200 ppm d'isoprène, de 10 à 100 ppm d'acétone et de 10 à 50 ppm de diéthylamine.

## Patentansprüche

1. Stabilisierte Lösung von trans-1,2-Dichlorethylen, umfassend als Additive mindestens:
- einen Säureakzeptor, der aus organischen Epoxiden ausgewählt ist,
- einen Radikalfänger, der aus einem Alken, einem Heterocyclus oder einem Phenolderivat ausgewählt ist,
- eine Lewis-Base, die aus einem Acetal, einem Keton, einer Nitrosoverbindung, einem Carbonsäureester oder einem Ether ausgewählt ist,
- und eine Verbindung mit Pufferwirkung, die aus N-Methylmorpholin, Diethylamin und Triethylamin ausgewählt ist.

2. Lösung nach Anspruch 1, **dadurch gekennzeichnet, dass** der Gehalt jedes Additivs in der stabilisierten Lösung zwischen 10 und 10.000 ppm und vorzugsweise zwischen 10 und 1000 ppm liegt.

3. Lösung nach Anspruch 1 oder 2, **dadurch gekennzeichnet, dass** der Gesamtgehalt an Additiven weniger als 50.000 ppm und vorzugsweise weniger als 5000 ppm beträgt.

4. Lösung nach einem der vorhergehenden Ansprüche, **dadurch gekennzeichnet, dass** sie 200 ppm bis 800 ppm eines Säureakzeptors, 100 bis 700 ppm eines Radikalfängers oder einer Mischung von Radikalfängern, 10 bis 100 ppm einer Lewis-Base und 10 bis 50 ppm einer Verbindung mit Pufferwirkung umfasst.

5. Lösung nach einem der vorhergehenden Ansprüche, **dadurch gekennzeichnet, dass** das organische Epoxid aus Propylenoxid, Butylenoxid oder Isopropylglycidylether ausgewählt ist und vorzugsweise Butylenoxid ist.

6. Lösung nach einem der vorhergehenden Ansprüche, **dadurch gekennzeichnet, dass** das Alken aus Diisobutylen, Amylen, Isopren und α-Methylstyrol ausgewählt ist.

7. Lösung nach einem der Ansprüche 1 bis 5, **dadurch gekennzeichnet, dass** es sich bei dem Radikalfänger um einen Heterocyclus, der aus N-Methylpyrrol, 1,4-Dioxan oder Tetrahydrofuran ausgewählt ist, handelt.

8. Lösung nach einem der Ansprüche 1 bis 5, **dadurch gekennzeichnet, dass** es sich bei dem Radikalfänger um ein Phenolderivat, das aus Phenol, Thymol oder Ionol ausgewählt ist, handelt.

9. Lösung nach einem der vorhergehenden Ansprüche, **dadurch gekennzeichnet, dass** es sich bei dem Acetal um Methylal handelt.

10. Lösung nach einem der Ansprüche 1 bis 8, **dadurch gekennzeichnet, dass** es sich bei dem Keton um Aceton oder Methylethylketon handelt.

11. Lösung nach einem der Ansprüche 1 bis 8, **dadurch gekennzeichnet, dass** es sich bei der Nitrosoverbindung um Nitromethan oder Nitroethan handelt.

12. Lösung nach einem der Ansprüche 1 bis 8, **dadurch gekennzeichnet, dass** es sich bei dem Carbonsäureester um Ameisensäuremethylester, Essigsäuremethylester oder Essigsäureisopropylester handelt.

13. Lösung nach einem der Ansprüche 1 bis 8, **dadurch gekennzeichnet, dass** es sich bei der Lewis-Base um einen Ether, vorzugsweise tert-Butylmethylether handelt.

14. Lösung nach einem der vorhergehenden Ansprüche, **dadurch gekennzeichnet, dass** es sich bei der Verbindung mit Pufferwirkung um N-Methylmorpholin handelt.

15. Lösung nach einem der Ansprüche 1 bis 13, **dadurch gekennzeichnet, dass** es sich bei der Verbindung mit Pufferwirkung um Diethylamin handelt.

16. Lösung nach einem der Ansprüche 1 bis 4, **dadurch gekennzeichnet, dass** sie Butylenoxid, Diisobutylen, Isopren, Aceton und Diethylamin umfasst.

17. Lösung nach Anspruch 16, **dadurch gekennzeichnet, dass** sie 200 bis 800 ppm Butylenoxid, 100 bis 500 ppm Diisobutylen, 50 bis 200 ppm Isopren, 10 bis 100 ppm Aceton und 10 bis 50 ppm Diethylamin umfasst.

## Claims

1. Stabilized solution of trans-1,2-dichloroethylene comprising, as additives, at least:
- one acid acceptor chosen from organic epoxides,
- one radical scavenger chosen from an alkene, a heterocycle or a phenol derivative,
- one Lewis base chosen from an acetal, a ketone, a nitro compound, an ester of a carboxylic acid or an ether,
- and one compound possessing a buffering effect chosen from N-methylmorpholine, diethylamine and triethylamine.

2. Solution according to Claim 1, **characterized in that** the content of each additive in the stabilized solution is between 10 and 10 000 ppm, preferably between 10 and 1000 ppm.

3. Solution according to Claim 1 or 2, **characterized in that** the overall content of additives is less than 50 000 ppm, preferably less than 5000 ppm.

4. Solution according to any one of the preceding claims, **characterized in that** it comprises from 200 ppm to 800 ppm of an acid acceptor, from 100 to 700 ppm of a radical scavenger or of a mixture of radical scavengers, from 10 to 100 ppm of a Lewis base and from 10 to 50 ppm of a compound possessing a buffering effect.

5. Solution according to any one of the preceding claims, **characterized in that** the organic epoxide is chosen from propylene oxide, butylene oxide or isopropyl glycidyl ether and is preferably butylene oxide.

6. Solution according to any one of the preceding claims, **characterized in that** the alkene is chosen from diisobutylene, amylene, isoprene or α-methylstyrene.

7. Solution according to any one of Claims 1 to 5, **characterized in that** the radical scavenger is a heterocycle chosen from N-methylpyrrole, 1,4-dioxane or tetrahydrofuran.

8. Solution according to any one of Claims 1 to 5, **characterized in that** the radical scavenger is a phenol derivative chosen from phenol, thymol or ionol.

9. Solution according to any one of the preceding claims, **characterized in that** the acetal is methylal.

10. Solution according to any one of Claims 1 to 8, **characterized in that** the ketone is acetone or methyl ethyl ketone.

11. Solution according to any one of Claims 1 to 8, **characterized in that** the nitro compound is nitromethane or nitroethane.

12. Solution according to any one of Claims 1 to 8, **characterized in that** the ester of a carboxylic acid is methyl formate, methyl acetate or isopropyl acetate.

13. Solution according to any one of Claims 1 to 8, **characterized in that** the Lewis base is an ether, preferably tert-butyl methyl ether.

14. Solution according to any one of the preceding claims, **characterized in that** the compound possessing a buffering effect is N-methylmorpholine.

15. Solution according to any one of Claims 1 to 13, **characterized in that** the compound possessing a buffering effect is diethylamine.

16. Solution according to one of Claims 1 to 4, **characterized in that** it comprises butylene oxide, diisobutylene, isoprene, acetone and diethylamine.

17. Solution according to Claim 16, **characterized in that** it comprises from 200 to 800 ppm of butylene oxide, from 100 to 500 ppm of diisobutylene, from 50 to 200 ppm of isoprene, from 10 to 100 ppm of acetone and from 10 to 50 ppm of diethylamine.
